# EUROPEAN PATENT APPLICATION

(11) **EP 0 958 818 A1**
(43) Date of publication of application: **24.11.1999**
(21) Application number: 97940341.7
(22) Date of filing: 11.09.1997
(51) Int. Cl.: A61K 31/425, C07D 417/12

(54) **GLUTATHIONE REDUCTASE ACTIVITY POTENTIATOR CONTAINING TROGLITAZONE**

(30) Priority: 12.09.1996 JP 24191096
(71) Applicant: SANKYO COMPANY LIMITED, Chuo-ku, Tokyo 103-0023 (JP)
(72) Inventor: YOKOYAMA, Tomihisa,Sankyo Company, Limited, Tokyo 140 (JP); FUJIWARA, Toshihiko,Sankyo Company, Limited, Tokyo 140 (JP); HORIKOSHI, Hiroyoshi,Sankyo Company, Limited, Tokyo 140 (JP); YOSHIOKA, Shinji,Sankyo Company, Limited, Tokyo 140 (JP)
(74) Representative: Gibson, Christian John Robert
(86) International application number: JP9703199
(87) International publication number: WO9810760

(57) **Abstract**

Problem: to provide a glutathione reductase activity potentiator. Means for resolution: a glutathione reductase potentiator containing troglitazone or a pharmacologically acceptable salt thereof.

## Description

### [Technical Field]

The present invention relates to an excellent agent for enhancing the activity of glutathione reductase comprising troglitazone or a pharmacologically acceptable salt thereof.

### [Background of the Invention]

Glutathione is found throughout the tissues of the living body, is a major reducing agent in cells, and plays a very important role in the oxidation-reduction metabolic processes. In particular, reduced glutathione (GSH), thanks to the presence of a thiol group, plays a key role in various cellular defence and repair mechanisms. Glutathione peroxidase is an enzyme which catalyses reaction wherein peroxides (such as hydrogen peroxide and lipoperoxide) are reduced by GSH, and is an important enzyme in the antioxidant system. On the other hand, glutathione reductase is an enzyme which reduces oxidized glutathione (oxidized-type glutathione: GSSG) into GSH in the presence of NADPH.

The antioxidant system comprising these materials and enzymes protects cells from the harmful effects of oxidising materials (e.g. above described peroxides, free radicals and so on). Oxidative stress occurs when the balance between oxidising materials and the antioxidant mechanisms is shifted in favor of the former [J. Appl. Physiol. Nov., 81(5), 2199-2202(1996)]. It has been reported that oxidative stress is associated with various diseases, such as coronary heart disease; cataracts; idiopathic pulmonary fibrosis; chronic renal failure; disorders of the nervous system including the peripheral nervous system and the central nervous system (e.g., Parkinson's disease, Alzheimer's disease, epilepsy, amyotrophic lateral sclerosis and cerebral ischemia); and gastric ulcers [J. Appl. Physiol. Nov., 81(5), 2199-2202(1996); Free Radical Biology & Medicine, 21(6), 845-853(1996); Free Radical Biology & Medicine, 20(7), 925-931(1996); and Gastroenterology, 112, 855-863(1997)]

In WO94/12527, it is disclosed that compounds which enhance the synthesis of endogenous GSH are suitable for human therapy, in particular for the treatment of various diseases induced by glutathione deficiency, such as the pathological states related to oxidative tissue damage, in particular when resulting from an excess of free radicals. Some examples of such diseases are: intracellular oxidative state disequilibrium following alcohol abuse, exposure to xenobiotic agents, damage caused by radiation, hepatic diseases, intoxication from drugs and chemical agents, poisoning by heavy metals, physiological brain ageing (e.g. Parkinson's disease, which is brain degeneration due to decreased glutathione levels caused by altered antioxidant defence mechanisms), acute and chronic neurodegenerative diseases (e.g., acute pathologies such as: acute ischemic states, in particular cerebral ictus, hypoglycemia, and epileptic attacks; chronic pathologies such as: amyotrophic lateral sclerosis, Alzheimer's disease, Huntington's chorea), diseases related to altered functionality of the immune system, in particular resulting from tumour immunotherapy, and infertility, in particular male infertility. It is also disclosed that the compounds are suitable for organ reperfusion following ischemic events mainly imputable to free radicals. In Japanese Patent Application Kokai No. Sho 64-26516, it is disclosed that a compound which increases glutathione levels is useful for the treatment and prevention of various diseases including cataracts; hepatic disorders and nephritic disorders.

From these disclosures, it is presumed that a compound which enhances glutathione reductase activity and increases supply of GSH exhibits similar effects to that of the above-described compound enhancing the synthesis of endogenous GSH or the compound increasing the levels of glutathione, and that it is therefore useful for the prevention or treatment of the diseases as exemplified above.

As described above, cataracts are also a condition in which oxidative stress takes part. It is reported that a dramatic decrease in GSH in the lens is observed in patients suffering from cataracts and that thiol groups of proteins have been oxidized at a high rate in the lenses of senile patients, patients suffering from X-ray cataracts and patients suffering from nuclear cataracts [V.N. Reddy, Exp. Eye Res. 50, 771-778(1990)]. Accordingly, it is presumed that these cataracts caused mainly by oxidative stress can be prevented or treated by enhancing glutathione reductase activity.

Diabetic cataracts are cataracts caused by diabetes and they are one of the diabetic complications. In patients suffering from diabetes, the polyol metabolic system is sthenic and this phenomenon is presumed to cause various diabetic complications including diabetic cataracts. In this polyol metabolic system, the reaction of reducing intracellular glucose into sorbitol by aldose reductase (AR) in the presence of NADPH is a rate-determining step so that the production amount of sorbitol increases by the sthenia of the polyol metabolic system [Satish K. Srivastava et al., Proc. Natl. Acad. Sci. 82, 7222-7226(1995)]. The sorbitol is accumulated in the cell because of low permeability across the biomembrane. The accumulation of sorbitol in the lens is presumed to increase osmotic pressure, swell the lens [Kinoshita et al., Metabolism 28-4 sup-1, 462-469(1979)], cause edema and denaturation of cell, resulting in the onset of a cataract. A number of AR inhibitors have been studied and developed on the basis of the hypothesis that cataracts can be prevented or treated by inhibiting AR, thereby controlling the intracellular accumulation of sorbitol.

It is, on the other hand, reported that based on the fact that when there exist both glutathione reductase and AR, the former one preferentially consumes NADPH, so that the enhancement of glutathione reductase activity makes it possible to suppress the function of AR, this results in the inhibition of the accumulation of a polyol [Yokoyama et al., Exp. Eye Res. 58, 207-218(1994)].

In short, it is presumable that diabetic cataract can be prevented or treated also by a compound which can enhance glutathione reductase activity.

Troglitazone is commercially available as a non insulin dependent antidiabetic agent and it is known to be useful for the treatment or prevention of various other diseases including diabetic complications. It is, however, not known that troglitazone has an ability to enhance the activity of glutathione reductase.

### [Disclosure of the Invention]

The present inventors have carried out an extensive investigation on the use of troglitazone as a medicament. As a result, it has been found that troglitazone has excellent effects for enhancing the activity of glutathione reductase.

The present invention provides:
(1) an agent for enhancing the activity of glutathione reductase, which comprises troglitazone or a pharmacologically acceptable salt thereof.
   In a preferred aspect of the present invention, there is also provided:
(2) an agent for enhancing the activity of glutathione reductase as described above in (1) for the prevention or treatment of diseases resulting from a deficiency of reduced glutathione.
   In a more preferred aspect of the present invention, there is also provided:
(3) an agent for enhancing the activity of glutathione reductase as described above in (1) for the prevention or treatment of diseases resulting from oxidative stress.
   In a still more preferred aspect of the present invention, there are also provided:
(4) an agent for enhancing the activity of glutathione reductase as described above in (1) for the prevention or treatment of coronary heart disease,
(5) an agent for enhancing the activity of glutathione reductase as described above in (1) for the prevention or treatment of idiopathic pulmonary fibrosis,
(6) an agent for enhancing the activity of glutathione reductase as described above in (1) for the prevention or treatment of chronic renal failure,
(7) an agent for enhancing the activity of glutathione reductase as described above in (1) for the prevention or treatment of disorders of the nervous system resulting from oxidative stress,
(8) an agent for enhancing the activity of glutathione reductase as described above in (1) for the prevention or treatment of gastric ulcers, and
(9) an agent for enhancing the activity of glutathione reductase as described above in (1) for the prevention or treatment of cataracts.
   In a particularly preferred aspect of the present invention as described above in (9), there is also provided:
(10) an agent for enhancing the activity of glutathione reductase as described above in (9), wherein said cataracts are caused by the accumulation of a polyol in the lens, the accumulation of a high molecular weight protein in the lens or the accumulation of a peroxide in the lens.
   In more preferred aspect of the present invention, there is also provided:
(11) an agent for enhancing the activity of glutathione reductase as described above in (9), wherein said cataracts are caused by the accumulation of a high molecular weight protein in the lens or the accumulation of a peroxide in the lens.
   In a more preferred aspect of the present invention, there are also provided:
(12) an agent for enhancing the activity of glutathione reductase as described above in (9), wherein said cataracts are senile cataracts.
   In the most preferred aspect of the present invention, there is also provided:
(13) an agent for enhancing the activity of glutathione reductase as described above in (9), wherein said cataracts are not diabetic cataracts but senile cataracts.

In the present invention,
the term "diseases resulting from a deficiency of reduced glutathione" means diseases such as intracellular oxidative state disequilibrium following alcohol abuse, exposure to xenobiotic agents, damage caused by radiation, hepatic diseases; intoxication from drugs and chemical agents; poisoning by heavy metals; disorders of the nervous system such as degenerative diseases of the brain and nervous system (e.g. cerebral ischemia, cerebral ictus, hypoglycemia, epilepsy, amyotrophic lateral sclerosis, Parkinson's disease, Alzheimer's disease, and Huntington's chorea); diseases related to altered functionality of the immune system, in particular resulting from tumour immunotherapy; infertility, in particular male infertility; coronary heart disease; cataracts; idiopathic pulmonary fibrosis; chronic renal failure; and gastric ulcers.

The term "diseases resulting from oxidative stress" as used herein means the above-exemplified "disorders of the nervous system", coronary heart disease, cataracts, idiopathic pulmonary fibrosis, chronic renal failure and gastric ulcers, and the term "nervous system disease resulting from oxidative stress" as used herein means the above-described "disorders of nervous system".

The term "polyol" as used herein means a sugar alcohol formed by the reduction of sugar with aldose reductase (AR) and examples of such a sugar alcohol include sorbitol and galactitol.

The term "cataracts caused by the accumulation of a polyol in the lens" as used herein means particularly diabetic cataracts, sugar cataracts and galactose cataracts.

The term "high molecular weight protein" as used herein means a protein obtained by forming a disulfide bond in the molecule or between molecules of a precursor peptide or protein which has in the molecule thereof at least one thiol group, through said thiol group. The term "peptide or protein which has in the molecule thereof at least one thiol group" as used herein embraces cell membrane proteins containing a thiol group which relates to cation carrying capacity and membrane permeability.

Examples of the "cataracts caused by the accumulation of a high molecular weight protein in the lens" include particularly senile cataracts, X-ray cataracts and nuclear cataracts.

Examples of the "peroxide" include oxidative free radicals such as super oxide (O₂⁻), a hydroxy radical (^{·}OH), singlet oxygen (^{·}O₂), a peroxy radical and an alkoxy radical; and compounds, which can release oxidative free radicals, such as hydrogen peroxide (H₂O₂) and lipoperoxide.

With reference to the "cataracts caused by the accumulation of peroxides in the lens", senile cataracts can be mentioned particularly.

Cataracts which are "not diabetic but senile" refer to cataracts which occur as a result of ageing but are not caused by diabetes, that is, the cataracts occuring in the senile who are not suffering from diabetes mellitus.

In some of the "diseases resulting from the deficiency of reduced glutathione", patients cannot be completely restored to the normal state once they suffer from such a disease and in such a case, the term "treatment" means the prevention or retardation of the progress of the pathologic condition.

Troglitazone is a compound represented by the following formula (I):

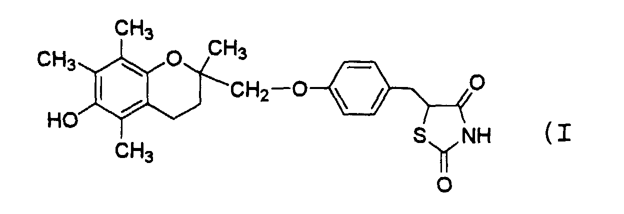

Since troglitazone can be obtained in the form of a salt, the term "pharmacologically acceptable salt" as used herein means such a salt. Preferred examples of such a salt include alkali metal salts such as the sodium salt, potassium salt and lithium salt and alkaline earth metal salts such as the calcium salt.

Troglitazone has two asymmetric carbons in the molecule thereof so there exist four isomers. In the present invention, these isomers and a mixture thereof are all expressed by one formula, that is the formula (1). The present invention therefore embraces all of these isomers and a mixture thereof.

When troglitazone or a pharmacologically acceptable salt thereof is allowed to stand in the air or recrystallized, it absorbs water or adsorbed water is attached to it and forms a hydrate. Such a hydrate is also embraced by the present invention.

The agent for enhancing the activity of glutathione reductase of the present invention comprises troglitazone or a pharmacologically acceptable salt thereof. Troglitazone can be prepared by the process as described in Japanese Patent Application Kokai No. Sho 61-51189.

Examples of the administration route of troglitazone or a pharmacologically acceptable salt thereof include oral administration in the form of tablets, capsules, granules, powders, syrups or the like and parenteral administration in the form of injection, suppository or the like. Such formulations are produced by known processes by using an additive such as excipients, lubricants, binders, disintegrating agents, stabilizers and corrigent.

Examples of the excipients include organic excipients, for example, sugar derivatives such as lactose, sucrose, glucose, mannitol or sorbitol; starch derivatives such as corn starch, potato starch, α-starch, dextrin or carboxymethyl starch; cellulose derivatives such as crystalline cellulose; gum arabic; dextran; and Pullulan; inorganic excipients including silicate derivatives such as light silicic acid anhydride, synthetic aluminium silicate or magnesium meta-silicic acid aluminate; phosphates such as calcium phosphate; carbonates such as calcium carbonate; and sulfates such as calcium sulfate.

Examples of the lubricants include stearic acid, metal stearates such as calcium stearate or magnesium stearate; talc; colloidal silica; waxes such as beeswax or spermaceti; boric acid; adipic acid; sulfates such as sodium sulfate; glycol; fumaric acid; sodium benzoate; DL-leucine; lauryl sulfates such as sodium lauryl sulfate or magnesium lauryl sulfate; silicates such as silicic acid anhydride or silicic acid hydrate; and the foregoing starch derivatives.

Examples of the binders include hydroxypropylcellulose, hydroxypropylmethylcellulose, polyvinylpyrrolidone, Macrogol and compounds similar to those exemplified above as the excipients.

Examples of the disintegrating agents include cellulose derivatives such as low-substituted hydroxypropylcellulose, carmellose, calcium carmellose, internally crosslinked sodium crosscarmellose; and chemically-modified starches/celluloses such as sodium carboxymethyl starch and cross-linked polyvinylpyrrolidone.

Examples of the stabilizers include paraoxybenzoate esters such as methylparaben or propylparaben; alcohols such as chlorobutanol, benzyl alcohol or phenylethyl alcohol; benzalkonium chloride; phenols such as phenol or cresol; thimerosal; dehydroacetic acid; and sorbic acid.

Examples of the corrigents include sweeteners, vinegar or perfumes such as those conventionally used.

The agent for enhancing the activity of glutathione reductase of the present invention comprising troglitazone can also be topically administered to eyes. Examples of formulations suitable for topical administration to the eye include a solid inserting form which remains in an almost perfect state even after administration and a disintegrative inserting form which is dissolved in tear fluid or disintegrates in another manner. Examples include solution, suspension, gel, ophthalmic ointment and solid inserting form.

The formulation of the ophthalmic composition may contain troglitazone or a pharmacologically acceptable salt thereof at a level of from 0.01% (preferably 0.1%) as a lower limit to 10% (preferably 5%) as an upper limit.

The ophthalmic composition may contain a non-toxic ophthalmic inorganic or organic carrier. Typical examples of the carrier for the ophthalmic composition include water; mixtures of water and a water-miscible solvent such as a lower alkanol or aralkanol; a vegetable oil; polyalkylene glycol; a jelly using petroleum as a base material; ethylcellulose; ethyl oleate; carboxymethylcellulose; polyvinylpyrrolidone; isopropyl myristate and other acceptable carriers which can be conventionally used. The ophthalmic composition may also contain non-toxic auxiliary substances such as an emulsifier, a preservative, a wetting agent and an excipient, for example, Polyethylene glycol 200, 300, 400 and 600, Carbowax 1000, 1500, 4000, 6000 and 10000, p-hydroxybenzoic acid esters such as methyl p-hydroxybenzoate or propyl p-hydroxybenzoate, a quaternary ammonium compound (for example, benzethonium chloride or benzalkonium chloride) which are known as compounds having anti-fungal properties at low temperatures and are non-toxic when used, an anti-fungal agent such as phenyl mercury salt, a buffering component such as thimerosal, methyl-and propylparaben, benzyl alcohol, phenylethanol, sodium chloride, sodium borate and sodium acetate, a gluconic acid buffering agent and sorbitan monolaurate, triethanolamine, polyoxyethylenesorbitan monopalmitate, sodium dioctyl sulfosuccinate, monothioglycerol, thiosorbitol and ethylenediaminetetraacetic acid.

Furthermore, the ophthalmic composition of the present invention may contain a proper ophthalmic excipient such as usual phosphoric acid buffering excipients (for example, a sodium phosphate buffer or potassium phosphate buffer), isotonic boric acid excipients, isotonic sodium chloride excipients and isotonic sodium borate excipients.

In the present invention, the dose of troglitazone or a pharmacologically acceptable salt thereof will vary depending on the condition and age of the patient, administration route and the like. However, for example, in the case of oral administration, for an adult human patient, it is desirable to administer from 0.1 mg (preferably 1 mg) as a lower limit to 1000 mg (preferably 500 mg) as an upper limit per day. In the case of intravenous administration, it is desirable to administer from 0.01 (preferably 0.1 mg) as a lower limit to 500 (preferably 200 mg) as an upper limit per day. It is administrated in single or divided doses per day depending on the condition of the patient.

The preparation containing troglitazone or a pharmacologically acceptable salt thereof can be prepared, for example, by the process as described below.

### Formulation Example 1: Powders

In a blender, 5 g of troglitazone, 895 g of lactose and 100 g of corn starch are mixed, whereby a powder can be obtained.

### Formulation Example 2: Granules

After mixing 5 g of troglitazone, 865 g of lactose and 100 g of low-substituted hydroxypropylcellulose, 300 g of a 10% aqueous solution, of hydroxypropylcellulose is added. The resulting mixture is then kneaded. The kneaded mass is granulated by an extruding granulator, followed by drying, whereby granules can be obtained.

### Formulation Example 3: Capsules

5 g of troglitazone, 115 g of lactose, 58 g of corn starch and 2 g of magnesium stearate are mixed using a V-type mixer. 180 mg of the resulting mixture are then encapsulated in a No.3 capsule to obtain a capsule formulation.

### Formulation Example 4: Tablets

5 g of troglitazone, 90 g of lactose, 34 g of corn starch, 20 g of crystalline cellulose and 1 g of magnesium stearate are mixed by means of a blender. The mixture is then pelletised by means of a tablet-making machine to obtain tablets.

### Formulation Example 5: Eyedrops

| | |
|---|---|
| Troglitazone | 2.0 g |
| Disodium phosphate | 0.716 g |
| Monosodium phosphate | 0.728 g |
| Sodium chloride | 0.400 g |
| Methyl p-hydroxybenzoate | 0.026 g |
| Propyl p-hydroxybenzoate | 0.014 g |
| Sterilized and purified water | q.s. |
| Sodium hydroxide | q.s. |
| Total | 100 ml |

The pH of the mixture is adjusted to 7.0 and eye drops are prepared by a conventional method.

### [Best Modes for Carrying Out the Invention]

The present invention will hereinafter be described in further detail with reference to examples. The examples however are not be construed as limiting the scope of the invention.

### Example 1: Effect enhancing the activity of reductase

### (1) Lens tissue culture

6 to 8 week old SD male rats (Japan SLC) were sacrificed by suffocation by inhalation of carbon dioxide. Both eyeballs of each test animal were excised. An incision was made in the sclera on the back of the eyeballs, and then the vitreous body and iris-ciliary body were removed, followed by removal of the lens.

Each lens obtained in this manner was cultured by immersing it in 3 ml of the culture solution described below in a 6-well tissue culture plate (FALCON). Culturing was performed for for 48 hours in a CO₂ incubator maintained at 37°C and 100% humidity in the presence of 5% CO₂ (in air).

As a culture solution for negative control, Medium 199 (GIBCO) containing penicillin (20 units/ml) and streptomycin (20 µg/ml) was used.

As a culture solution for positive control was employed a solution containing galactose (D-galactose) at a concentration of 30 mM which solution had been prepared by dissolving galactose in a mixed solution (85:15) of the above-described culture solution for negative control and purified water.

A culture solution for test was prepared by dissolving or suspending troglitazone in 10% aqueous acetone and it was then added to the above-described culture solution for positive control in an amount of 1% to the resulting solution or suspension. The culture solutions for test containing troglitazone in concentrations of 0.2 µM, 2 µM and 20 µM were prepared and used.

After incubation, the lens was frozen for storage.

The lens incubated in the culture solution for negative control corresponds to the lens under the normal state, the lens incubated in the culture solution for positive control corresponds to the lens under galactose load and the lens incubated in the culture solution for test corresponds to the troglitazone-administered lens.

### (2) Measurement of the activity of glutathione reductase

An aqueous solution of glutathione reductase was prepared using the lens incubated in (1) and its reductase activity was measured.

Described specifically, the frozen rat lens was homogenized in 2 ml of distilled water, followed by separation by centrifugation (10,000 g, for 20 minutes). The resulting supernatant was employed as the enzyme sample.

400 µl of the enzyme sample were added to 0.6 ml of a phosphate buffer containing 1 mM oxidized glutathione (GSSG) and 100 µM NADPH. After the mixture had reacted at 25°C for 6 minutes, the absorbance of NADPH (340 nm: OD₃₄₀ₙₘ) which had remained in the reaction mixture without consumption was measured. The difference (ΔOD₃₄₀ₙₘ) between the OD₃₄₀ₙₘ value before reaction and OD₃₄₀ₙₘ value after completion of reaction was used as an indicator of the consumption amount of NADPH and used as an index of glutathione reductase activity. Results (ΔOD₃₄₀ₙₘ/minute/lens) are shown in Table 1.

**Table 1**

| Control group | | Troglitazone added group | |
|---|---|---|---|
| Negative control (- galactose) | Positive control (+ galactose) | 2 µM | 20 µM |
| 0.027±0.001 | 0.026±0.001 | 0.031±0.000 | 0.031±0.001 |

As is apparent from Table 1, the ΔOD₃₄₀ₙₘ value of the troglitazone-added group is larger, that is, the consumption amount of NADPH is greater than those of the negative and positive control tests, which indicates that the addition of troglitazone enhanced the glutathione reductase activity.

### Example 2: Inhibitory action on aldose reductase (AR)

AR activity was measured in a similar method to that described by Hayman and Kinoshita et al. (J. Biol. Chem. 240, 877-882(1965)) by using the lens cultured according to Example 1(1) as the origin of the enzyme.

Described specifically, the lens cultured according to Example 1(1) was washed with physiological saline and homogenized in a 0.05 mM EDTA solution. The homogenate was subjected to centrifugal separation (10,000 g, for 15 minutes) and the resulting supernatant was used as an enzyme sample. The resulting enzyme sample was added to a solution of 0.5 mM DL-glyceraldehyde, 0.4 M lithium sulfate, 0.05 mM NADPH, 67 mM sodium monohydrogen phosphate and 1 mM EDTA in distilled water to initiate enzymatic reaction. After reaction at room temperature for 7 minutes, the absorbance (340 nm) of NADPH which had remained in the reaction mixture without consumption was measured. The difference between the absorbance before the reaction and that after completion of reaction as an NADPH consumption amount was used as an index of AR activity.

Troglitazone did not exhibit AR inhibitory action in the concentration of 0.2 µM or 2 µM.

### Example 3: Inhibitory action on the accumulation of polyol in the lens

The amount of galactitol, which is a polyol prepared through the reduction of galactose by AR in the lens was measured.

Described specifically, the lens cultured and frozen according to Example 1(1) was homogenized in 1 ml of a 0.6 mM dimethylmercaptoglucose (internal standard substance) solution in distilled water. 2.4 ml of ice-cooled ethanol was added to the homogenate and the resulting mixture was allowed to stand for 5 minutes. The reaction mixture was then subjected to centrifugation (10,000 g, for 5 minutes) and 25 µl of the resulting supernatant was put into a screw-cap vial, followed by lyophilization. After the addition of 70 µl of pyridine and 20 µl of phenyl isocyanate to the lyophilization product, the resulting mixture was incubated at 55°C for 5 minutes. Then 20 µl of methanol was added and the resulting mixture was incubated at 55°C for one hour. To the incubated mixture, 90 µl of pyridine was added to prepare a specimen. The specimen was injected into a high-performance liquid chromatography apparatus (eluent: a 5:2:3 mixture of acetonitrile : ethanol : water, flow rate: 1 ml/mm). Its absorbance (240 nm) was measured by a spectrophotometer and the amount of galactitol contained in each lens was calculated. Shown in Table 2 is the amount of galactitol in the troglitazone-added group when the amount of galactitol in the positive control group is designated as 100.

**Table 2**

| Concentration of troglitazone added | 0 µM (positive control) | 2 µM | 20 µM |
|---|---|---|---|
| Amount of galactitol | 100 | 65±8 | 47±6 |

As is apparent from Table 2, troglitazone inhibited the accumulation of galactitol in the lens depending on the concentration of it.

### Example 4: Effect in lowering lipoperoxide

The amount of lipoperoxide in the lens of a rat cultured in accordance with Example 1(1) was measured.

Described specifically, after the measurement of the wet weight of the lens, it was homogenized in 0.5 ml of a 50 mM tris hydrochloride buffer (pH 7.6) containing 1 mM EDTA. The homogenate was subjected to centrifugation (2,500 g, for 30 minutes) and the lipoperoxide contained in the supernatant was determined by a kit for measuring lipoperoxide ("LPO-586", BIOXYTECH S.A.). The colorimetry of the lipoperoxide was carried out with malonaldehyde and hydroxyalkenal as an index of lipoperoxide. The results (amount of lipoperoxide in 1 g of the lens: nmol/g) are shown in Table 3.

**Table 3**

| Control group | | Troglitazone added group | |
|---|---|---|---|
| Negative control (- galactose) | Positive control (+ galactose) | 2 µM | 20 µM |
| 0.68±0.28 | 5.17±1.09 | 4.52±0.19 | 1.25±0.92 |

As is apparent from Table 3, the concentration of lipoperoxide in the lens increased due to applying load by galactose, but the degree of the increase is lowered by the addition of troglitazone depending on the concentration of troglitazone.

### [Possibility of Industrial Use]

As shown in Example 1, since troglitazone exhibits an effect of enhancing the activity of glutathione reductase and can increase the amount of GSH to be supplied in the living body, it is useful for the prevention or treatment of diseases resulting from the deficiency of reduced glutathione. In addition, as shown in Example 4, troglitazone can decrease the amount of lipoperoxide so that it is usable for the prevention or treatment of diseases resulting from oxidative stress (particularly, cataracts which are a condition resulting from oxidative stress in the lens).

Furthermore, from the results of Examples 1, 2 and 3, it is apparent that troglitazone exhibited an effect of enhancing the activity of glutathione reductase activity and inhibitory activity against polyol accumulation even at the concentration (2 µM) at which it does not exhibit AR inhibitory activity. Namely, troglitazone inhibited the polyol metabolic system by enhancing the activity of glutathione reductase, thereby suppressing the accumulation of polyol. Accordingly, the agent for enhancing the activity of glutathione reductase comprising troglitazone can be used for the prevention or treatment of diseases caused by the intracellular accumulation of a polyol such as diabetic cataracts.

## Claims

1. An agent for enhancing the activity of glutathione reductase, which comprises troglitazone or a pharmacologically acceptable salt thereof.

2. An agent for enhancing the activity of glutathione reductase described in claim 1 for the prevention or treatment of diseases resulting from a deficiency of reduced glutathione.

3. An agent for enhancing the activity of glutathione reductase described in claim 1 for the prevention or treatment of diseases resulting from oxidative stress.

4. An agent for enhancing the activity of glutathione reductase described in claim 1 for the prevention or treatment of coronary heart disease.

5. An agent for enhancing the activity of glutathione reductase described in claim 1 for the prevention or treatment of idiopathic pulmonary fibrosis.

6. An agent for enhancing the activity of glutathione reductase described in claim 1 for the prevention or treatment of chronic renal failure.

7. An agent for enhancing the activity of glutathione reductase described in claim 1 for the prevention or treatment of disorders of the nervous system resulting from oxidative stress.

8. An agent for enhancing the activity of glutathione reductase described in claim 1 for the prevention or treatment of gastric ulcers.

9. An agent for enhancing the activity of glutathione reductase described in claim 1 for the prevention or treatment of cataracts.

10. An agent for enhancing the activity of glutathione reductase described in claim 9, wherein said cataracts are caused by the accumulation of a polyol in the lens, the accumulation of a high molecular weight protein in the lens or the accumulation of a peroxide in the lens.

11. An agent for enhancing the activity of glutathione reductase described in claim 9, wherein said cataracts are caused by the accumulation of a high molecular weight protein in the lens or the accumulation of a peroxide in the lens.

12. An agent for enhancing the activity of glutathione reductase described in claim 9, wherein said cataracts are senile cataracts.

13. An agent for enhancing the activity of glutathione reductase described in claim 9, wherein said cataracts are not diabetic cataracts but senile cataracts.
